(19) Europäisches Patentamt
European Patent Office
Office européen des brevets

(11) EP 2 179 745 A1

(12) **EUROPEAN PATENT APPLICATION**

(43) Date of publication:
**28.04.2010 Bulletin 2010/17**

(21) Application number: **09178302.7**

(22) Date of filing: **06.06.2003**

(51) Int Cl.:
***A61K 45/06** (2006.01)* ***A61K 39/395** (2006.01)*
***A61K 31/426** (2006.01)* ***A61K 31/427** (2006.01)*
***A61P 35/00** (2006.01)*

(84) Designated Contracting States:
**AT BE BG CH CY CZ DE DK EE ES FI FR GB GR HU IE IT LI LU MC NL PT RO SE SI SK TR**

(30) Priority: **10.06.2002 US 387025 P**
**27.08.2002 US 406238 P**
**27.08.2002 US 406239 P**
**08.01.2003 US 438677 P**
**08.01.2003 US 438676 P**

(62) Document number(s) of the earlier application(s) in accordance with Art. 76 EPC:
**03757040.5 / 1 515 750**

(71) Applicants:
- **Novartis AG**
  **4056 Basel (CH)**
  Designated Contracting States:
  **BE BG CH LI CY CZ DE DK EE ES FI FR GB GR HU IE IT LU MC NL PT RO SE SI SK TR**
- **Novartis Pharma GmbH**
  **1230 Wien (AT)**
  Designated Contracting States:
  **AT**

(72) Inventors:
- **Hohneker, John Arthur**
  **Morristwon, NJ 07960 (US)**
- **Miller, Julie Ann**
  **Whippany, NJ 07981 (US)**
- **Rothermel, John David**
  **Randolph, NJ 07869 (US)**
- **Wartmann, Markus**
  **4125 Riehen (CH)**

(74) Representative: **de Weerd, Petrus G.W. et al**
**Novartis Pharma AG**
**Patent Department**
**Lichtstrasse 35**
**4002 Basel (CH)**

Remarks:
This application was filed on 08-12-2009 as a divisional application to the application mentioned under INID code 62.

(54) **Combinations comprising epothilones and pharmaceutical uses thereof**

(57) The invention relates to a pharmaceutical combination which comprises (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; and (c) an epothilone derivative of formula I

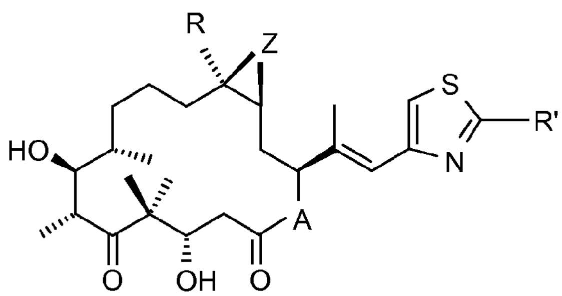

(I)

and optionally at least one pharmaceutically acceptable carrier for simultaneous, separate or sequential use, in particular,

EP 2 179 745 A1

for the treatment of a proliferative disease, especially a solid tumor disease; a pharmaceutical composition comprising such a combination; the use of such a combination for the preparation of a medicament for the treatment of a proliferative disease; a commercial package or product comprising such a combination as a combined preparation for simultaneous, separate or sequential use; and to a method of treatment of a warm-blooded animal, especially a human.

## Description

The invention relates to a pharmaceutical combination which comprises (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; and (c) an epothilone derivative and optionally at least one pharmaceutically acceptable carrier for simultaneous, separate or sequential use, in particular, for the treatment of a proliferative disease, especially a solid tumor disease; a pharmaceutical composition comprising such a combination; the use of such a combination for the preparation of a medicament for the treatment of a proliferative disease; a commercial package or product comprising such a combination as a combined preparation for simultaneous, separate or sequential use; and to a method of treatment of a warm-blooded animal, especially a human.

Despite the widespread use of Taxol® and Taxotere® in the treatment of many different tumor types, the impact of taxanes on patient survival has been modest, and the overwhelming majority of metastatic solid tumors remain incurable. Taxane treatment is associated with a number of significant side-effects, and the effectiveness of taxanes can be severely limited by the rapid development of drug resistance mechanisms. In view of these limitations as well as the side-effects commonly observed with standard combination therapies, there is a clear need for the identification of novel cytotoxic anti-cancer agents exhibiting an improved overall profile including spectrum of anti-tumor activity, efficacy against multi-drug resistant tumors, safety and tolerability.

The microtubule-stabilizing effect of the epothilones was first described by Bollag et al., Cancer Research 55, 1995, 2325-33. A suitable treatment schedule for the treatment of different types of tumors, especially tumors which are refractory to the treatment by other chemotherapeutics, in particular TAXOL™, using an epothilone, in particular epothilone A or B, is described in WO 99/43320.

Surprisingly, it has now been found that the anti-proliferative effect of a combination as defined herein is greater than the effect that can be achieved with either type of combination partner alone, i.e. greater than the effect of a monotherapy using only one of the combination partners (a) and (c) as defined herein.

Surprisingly, it has been found that the antineoplastic effect, in particular in the treatment of a proliferative disease, especially a solid tumor disease, that is refractory to other chemotherapeutics known as antineoplastic agents, of a combination as defined herein is greater than the effects that can be achieved with either type of combination partner alone, i.e., greater than the effect of a monotherapy using only one of the combination partners (b) and (c) as defined herein.

Hence, the present invention pertains to a combination, such as a combined preparation or a pharmaceutical composition, which comprises (a) a HER-1 or a HER-2 antibody or (b) at least one antineoplastic agent selected from the group consisting of aromatase inhibitors antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors, and (c) an epothilone derivative of formula I

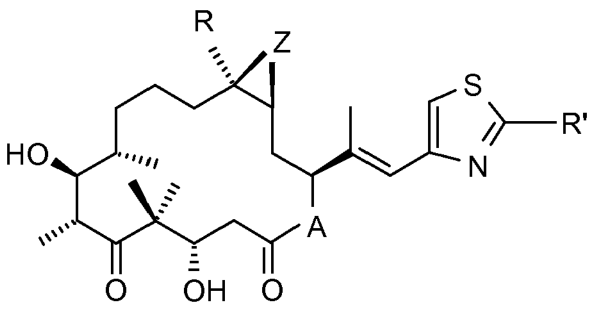

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O or a bond, in which the active ingredients (a) and (b) are present in each case in free form or in the form of a pharmaceutically acceptable salt and optionally at least one pharmaceutically acceptable carrier; for simultaneous, separate or sequential use.

Furthermore, the present invention relates to a method of treating a warm-blooded animal having a proliferative disease comprising administering to the animal a combination which comprises (a) a HER-1 or a HER-2 antibody or (b)

at least one antineoplastic agent selected from the group consisting of aromatase inhibitors antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors, and (c) an epothilone derivative of formula I wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O or a bond, in a quantity which is jointly therapeutically effective against a proliferative disease and in which the compounds can also be present in the form of their pharmaceutically acceptable salts.

Unless stated otherwise, in the present disclosure organic radicals and compounds designated "lower" contain not more than 7, preferably not more than 4, carbon atoms.

A compound of formula I wherein A represents O, R is hydrogen and Z is O is known as epothilone A; a compound of formula I wherein A represents O, R is methyl and Z is O is known as epothilone B; a compound of formula I wherein A represents O, R is hydrogen and Z is a bond is known as epothilone C; a compound of formula I wherein A represents O, R is methyl and Z is a bond is known as epothilone D.

The compounds used as combination partners (a), (b) and (c) disclosed herein can be prepared and administered as described in the cited documents, respectively, if not mentioned otherwise.

Epothilone derivatives of formula I wherein A represents O or $NR_N$, wherein $R_N$ is hydrogen or lower alkyl, R is hydrogen or lower alkyl and Z is O or a bond, and methods for the preparation of such epothilone derivatives are in particular generically and specifically disclosed in the patents and patent applications WO 93/10121, US 6,194,181, WO 98/25929, WO 98/08849, WO 99/43653, WO 98/22461 and WO 00/31247 in each case in particular in the compound claims and the final products of the working examples, the subject-matter of the final products, the pharmaceutical preparations and the claims is hereby incorporated into the present application by reference to this publications.

Comprised are likewise the corresponding stereoisomers as well as the corresponding crystal modifications, e.g. solvates and polymorphs, which are disclosed therein.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I wherein $R_N$ is lower alkyl can be prepared by methods known in the art such as a reductive alkylation reaction starting from the epothilone derivative wherein $R_N$ is hydrogen.

Epothilone derivatives of formula I wherein A represents O or $NR_N$, wherein $R_N$ is hydrogen or lower alkyl, R is hydrogen or lower alkyl, R' is methoxy, ethoxy, amino, methylamino, dimethylamino, aminomethyl or methylthio, and Z is O or a bond, and methods for the preparation and administration of such epothilone derivatives are in particular generically and specifically disclosed in the patent application WO99/67252, which is hereby incorporated by reference. Comprised are likewise the corresponding stereoisomers as well as the corresponding crystal modifications, e.g. solvates and polymorphs, which are disclosed therein.

A compound of formula I, wherein A represents O, R is methyl, R' is aminomethyl and Z is O can be prepared as described in Examples 13 to 16 of WO01/72721 starting with a compound of formula I, wherein A represents O, R and R' are both methyl and Z is O.

The term "a combined preparation", as used herein defines especially a "kit of parts" in the sense that the combination partners (a), (b) and (c) as defined above can be dosed independently or by use of different fixed combinations with distinguished amounts of the combination partners (a), (b) and (c), i.e., simultaneously or at different time points. The parts of the kit can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a), (b) and (c) or such that the effect of a combination partner (c) is potentiated due to the presence of a combination partner (a). The ratio of the total amounts of the combination partner (a) or (b) to the combination partner (c) to be administered in the combined preparation can be varied, e.g. in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, potentiation, i.e. a combined therapeutical effect in a non-effective dosage of one or both of the combination partners (a), (b) and (c), and very preferably a strong synergism of the combination partners (a), (b) and (c).

The term "solid tumor disease" as used herein comprises, but is not restricted to glioma, thyroid cancer, breast cancer, especially breast tumors positive for the overexpression of HER2/*neu*, ovarian cancer, cancer of the colon and generally the GI tract, cervix cancer, lung cancer, in particular small-cell lung cancer, and non-small-cell lung cancer, head and neck cancer, bladder cancer, cancer of the prostate, hepatoma or Kaposi's sarcoma. Depending on the tumor type and the particular combination used, a decrease of the tumor volume can be obtained. The combinations disclosed herein are also suited to prevent the metastatic spread of tumors and the growth or development of micrometastases.

The term "proliferative disease" comprises a solid tumor disease, a liquid tumor, e.g. leukemia, and psoriasis.

The term "solid tumor disease" especially means breast cancer, cancer of the colon and generally the GI tract including gastric cancer, hepatoma; lung cancer, in particular small-cell lung cancer and non-small-cell lung cancer, renal cancer, mesothelioma, glioma, squamous cell carcinoma of the skin, head and neck cancer, genitourinary cancer, e.g. cervical, uterine, ovarian, testicles, prostate or bladder cancer; Hodgkin's disease, carcinoid syndrome or Kaposi's sarcoma. In a preferred embodiment of the invention, the solid tumor disease to be treated is selected from breast cancer, colorectal cancer, ovarian cancer, renal cancer, lung cancer, especially non-small-cell lung cancer, and glioma. Depending on the tumor type and the particular combination used a decrease of the tumor volume can be obtained. The combinations disclosed herein are also suitable to prevent the metastatic spread of tumors and the growth or development of micrometastases.

The term "microtubule active agents" relates to microtubule stabilizing and microtubule destabilizing agents selected from the group consisting of the vinca alkaloids, e.g., vinblastine, especially vinblastine sulfate, vincristine especially vincristine sulfate, and vinorelbine and discodermolide. Vinblastine sulfate can be administered, e.g., in the form as it is marketed, e.g. under the trademark VINBLASTIN R.P.™. Vincristine sulfate can be administered, e.g., in the form as it is marketed, e.g. under the trademark FARMISTIN™. Discodermolide can be obtained, e.g., as disclosed in U.S. patent nos. 4,939,168 and 5,618,487 to Harbor Branch Oceanographic Institute or by chemical synthesis as described, for example, in GB 2280677, WO 98/24429 and U.S. patent nos. 5,789605 and 6,031,133, which are here incorporated by reference.

The term "protein kinase C inhibitors", refers in particular to staurosporine derivatives, and preferably to those disclosed in US 5,093,330. Such compounds can be administered, e.g., in the form as disclosed in WO99/48896.

The term "anti-angiogenic compounds" as used herein relates to thalidomide (THALOMID™) and SU5416.

The term "antiestrogens" as used herein relates to compounds which antagonize the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX™. Raloxifene hydrochloride can be administered, e.g., in the form as it is marketed, e.g. under the trademark EVISTA™. Fulvestrant can be formulated as disclosed in US 4,659,516 or it can be administered, e.g., in the form as it is marketed, e.g. under the trademark FASLODEX™.

The term "HER-1 or a HER-2 antibody" as used herein means monoclonal antibodies binding to the HER-1 and HEr-2 receptor, in particular the HER-2 receptor. Preferred are the antibodies which are generically and specifically disclosed in US 5,677,171, in particular, in the working examples hereby incorporated into the present application by reference to this publication. Comprised are likewise the corresponding stereoisomers as well as the corresponding crystal modifications, e.g. solvates and polymorphs, which are disclosed therein. More preferred is trastuzumab. The latter compound is also marketed under the tradename Herceptin™.

The term "topoisomerase I inhibitors" as used herein includes, but is not limited to topotecan, irinotecan, 9-nitrocamptothecin and the macromolecular camptothecin conjugate PNU-166148 (compound A1 in WO99/17804). Irinotecan can be administered, e.g., in the form as it is marketed, e.g. under the trademark CAMPTOSAR™. Topotecan can be administered, e.g., in the form as it is marketed, e.g. under the trademark HYCAMTIN™.

The term "topoisomerase II inhibitors" as used herein includes, but is not limited to the antracyclines doxorubicin (including liposomal formulation, e.g. CAELYX™), epirubicin, idarubicin and nemorubicin, the anthraquinones mitoxantrone and losoxantrone, and the podophillotoxines etoposide and teniposide. Etoposide can be administered, e.g., in the form as it is marketed, e.g. under the trademark ETOPOPHOS™. Teniposide can be administered, e.g., in the form as it is marketed, e.g. under the trademark VM 26-BRISTOL ™. Doxorubicin can be administered, e.g., in the form as it is marketed, e.g. under the trademark ADRIBLASTIN™. Epirubicin can be administered, e.g., in the form as it is marketed, e.g. under the trademark FARMORUBICIN™. Idarubicin can be administered, e.g., in the form as it is marketed, e.g. under the trademark ZAVEDOS™. Mitoxantrone can be administered, e.g., in the form as it is marketed, e.g. under the trademark NOVANTRON™.

The term "aromatase inhibitors" as used herein relates to compounds which inhibit the estrogen production, i.e. the conversion of the substrates androstenedione and testosterone to estrone and estradiol, respectively. The term includes, but is not limited to steroids, especially exemestane and formestane and, in particular, non-steroids, especially aminoglutethimide, vorozole, fadrozole, anastrozole and, very especially, letrozole. Exemestane can be administered, e.g., in the form as it is marketed, e.g. under the trademark AROMASIN™. Formestane can be administered, e.g., in the form as it is marketed, e.g. under the trademark LENTARON™. Fadrozole can be administered, e.g., in the form as it is marketed, e.g. under the trademark AFEMA™. Anastrozole can be administered, e.g., in the form as it is marketed, e.g. under the trademark ARIMIDEX™. Letrozole can be administered, e.g., in the form as it is marketed, e.g. under the trademark FEMARA™ or FEMAR™. Aminoglutethimide can be administered, e.g., in the form as it is marketed, e.g. under the trademark ORIMETEN™.

The term "gonadorelin agonist" as used herein includes, but is not limited to abarelix, goserelin and goserelin acetate. Goserelin is disclosed in US 4,100,274 and can be administered, e.g., in the form as it is marketed, e.g. under

the trademark ZOLADEX™. Abarelix can be formulated, e. g. as disclosed in US 5,843,901.

The term "anti-androgens" as used herein includes, but is not limited to bicalutamide (CASODEX™), which can be formulated, e.g. as disclosed in US 4,636,505.

The term "histone deacetylase inhibitors" as used herein includes, but is not limited to MS-275, SAHA, FK228 (formerly FR901228), Trichostatin A and the compounds disclosed in WO 02/22577, in particular NVP-LAQ824 or its lactate salt.

The term "S-adenosylmethionine decarboxylase inhibitors" as used herein includes, but is not limited to the compounds disclosed in US 5,461,076.

The structure of the active ingredients identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active ingredients and, based on these references, likewise enabled to manufacture and test the pharmaceutical indications and properties in standard test models, both *in vitro* and *in vivo.*

It will be understood that references to the combination partners (a), (b) and (c) are meant to also include the pharmaceutically acceptable salts. If these combination partners (a) and (b) have, for example, at least one basic center, they can form acid addition salts. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization.

The compounds used as combination partners (a) and (b) disclosed herein can be prepared and administered as described in the cited documents, respectively. Epothilone derivatives of formula I, especially epothilone B, can be administered as part of pharmaceutical compositions which are disclosed in WO 99/39694.

A combination of the invention comprising an antineoplastic agent which is an aromatase inhibitor is particularly useful for the treatment of hormone receptor positive breast tumors.

Preferred combinations are, in particular, those which comprises a HER-1 or a HER-2 antibody and an epothilone derivative.

Other preferred combinations are, in particular, those wherein the antineoplastic agent is a aromatase inhibitors or an antiestrogens.

Other preferred combinations are, in particular, those wherein the antineoplastic agent is a topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors.

Preferred combinations are, in particular, those wherein the antineoplastic agent is a topoisomerase I inhibitor, e.g. irinotecan, a topoisomerase II inhibitor, e.g. etoposide, or a microtubule active agent, e.g. discodermolide, aromatase inhibitor, e.g. letrozole.

Even more preferred combinations are combinations wherein the HER-1 or HER-2 antibody is trastuzumab.

Another even more preferred combinations are combinations wherein the antineoplastic agent is a topoisomerase I inhibitor.

A further even more preferred combinations are combinations wherein the antineoplastic agent is a topoisomerase II inhibitor.

Yet another even more preferred combinations are combinations wherein the antineoplastic agent is an aromatase inhibitor.

Yet another even more preferred combinations are combinations wherein the antineoplastic agent is a microtubule active agent.

A Preferred embodiment provides a combination which comprises

(a) a HER-1 or a HER-2 antibody and
(b) an epothilone derivative of formula I

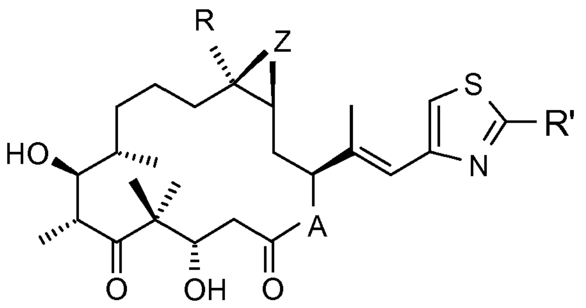

(I)

in which compound A represents O or $NR_N$, wherein $R_N$ is hydrogen or lower alkyl, R is hydrogen or lower alkyl, R' is methyl and Z is O or a bond,
in which the active ingredients (a) and (b) are present in each case in free form or in the form of a pharmaceutically acceptable salt and optionally at least one pharmaceutically acceptable carrier; for simultaneous, separate or sequential use.

Another preferred embodiment provides a combination which comprises (a) at least one antineoplastic agent selected from the group consisting of topoisomerase I inhibitors, topoisomerase II inhibitors, microtubule active agents, protein kinase C inhibitors, anti-angiogenic compounds, gonadorelin agonists, anti-androgens, histone deacetylase inhibitors, and S-adenosylmethionine decarboxylase inhibitors and (b) an epothilone derivative of formula I

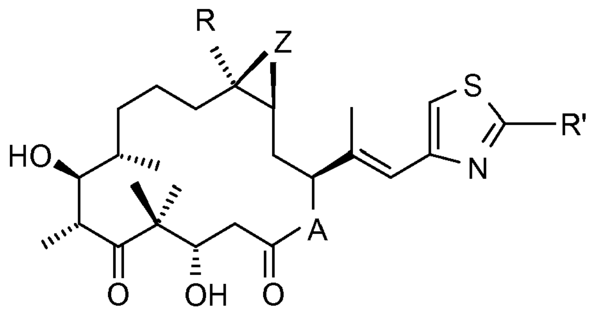

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O or a bond,
in which the active ingredients (a) and (b) are present in each case in free form or in the form of a pharmaceutically acceptable salt and optionally at least one pharmaceutically acceptable carrier; for simultaneous, separate or sequential use.

Another preferred embodiment provides a combination which comprises (a) at least one antineoplastic agent selected from the group consisting of aromatase inhibitors and antiestrogens and (b) an epothilone derivative of formula I

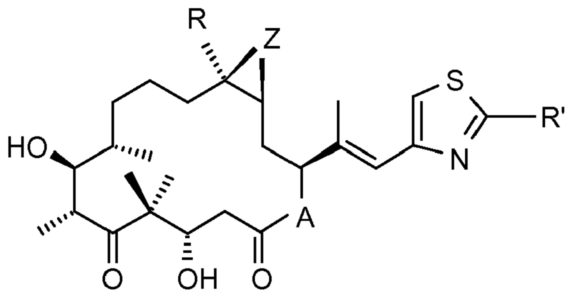

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O or a bond, in which the active ingredients (a) and (b) are present in each case in free form or in the form of a pharmaceutically acceptable salt and optionally at least one pharmaceutically acceptable carrier; for simultaneous, separate or sequential use.

Another preferred embodiment provides combination which comprises (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; and (c) an epothilone derivative of formula I wherein A represents $NR_N$, wherein $R_N$ is hydrogen or lower alkyl, or, more preferably O, R is hydrogen or lower alkyl, most preferably methyl, R' is methyl or methylthio, and Z is a bond, or, preferably, O., in which the active ingredients are present in each case in free form or in the form of a pharmaceutically acceptable salt and optionally at least one pharmaceutically acceptable carrier; for simultaneous, separate or sequential use.

An even more preferred embodiment provides combination which comprises (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; and (c) an epothilone derivative of formula I wherein A represents $NR_N$, wherein $R_N$ is hydrogen or lower alkyl, or, more preferably O, R is hydrogen or lower alkyl, most preferably methyl, R' is methyl, and Z is a bond, or, preferably, O., in which the active ingredients are present in each case in free form or in the form of a pharmaceutically acceptable salt and optionally at least one pharmaceutically acceptable carrier; for simultaneous, separate or sequential use.

Another even more preferred embodiment provides combination which comprises (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; and (c) an epothilone derivative of formula I wherein A represents $NR_N$, wherein $R_N$ is hydrogen or lower alkyl, or, more preferably O, R is hydrogen or lower alkyl, most preferably methyl, R' is methylthio, and Z is a bond, or, preferably, O., in which the active ingredients are present in each case in free form or in the form of a pharmaceutically acceptable salt and optionally at least one pharmaceutically acceptable carrier; for simultaneous, separate or sequential use.

An even more preferred embodiment is a combination which comprises (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; and (c) epothilone B, in which the active ingredients are present in each case in free form or in the form of a pharmaceutically acceptable salt and optionally at least one pharmaceutically acceptable carrier; for simultaneous, separate or sequential use.

The nature of proliferative diseases like solid tumor diseases is multifactorial. Under certain circumstances, drugs with different mechanisms of action may be combined. However, just considering any combination of drugs having different mode of action does not necessarily lead to combinations with advantageous effects.

The subject-matter of the final products, the pharmaceutical preparations and the claims of the patent rights cited herein-above is hereby incorporated into the present application by reference. Comprised are likewise the corresponding stereoisomers as well as the corresponding crystal modifications, e.g. solvates and polymorphs, which are disclosed therein. The compounds used as active ingredients in the combinations disclosed herein can be prepared and administered as described in the cited documents, respectively, if not otherwise mentioned herein.

All the more surprising is the experimental finding that *in vivo* the administration of a COMBINATION OF THE INVENTION, results not only in a beneficial effect, especially a synergistic therapeutic effect, e.g. with regard to slowing down, arresting or reversing the neoplasm formation or a longer duration of tumor response, but also in further surprising beneficial effects, e.g. less side-effects, an improved quality of life and a decreased mortality and morbidity, compared to a equiefficacious monotherapy applying only one of the pharmaceutically active ingredients used in the COMBINATION OF THE INVENTION, in particular in the treatment of a tumor that is refractory to other chemotherapeutics known as anti-cancer agents.

A further benefit is that lower doses of the active ingredients of the COMBINATION OF THE INVENTION can be used, for example, that the dosages of at least one combination partner need not only often be smaller, but are also applied less frequently, in order to diminish the incidence of side-effects. This is in accordance with the desires and requirements of the patients to be treated.

It can be shown by established test models and in particular those test models described herein that a COMBINATION OF THE INVENTION results in a more effective delay of progression or treatment of a proliferative disease compared to the effects observed with the single combination partners. The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

Suitable clinical studies are in particular open label non-randomized, dose escalation studies or placebo-controlled, double-blind studies in patients with advanced solid tumors. Such studies prove in particular the synergism of the active ingredients of the COMBINATIONS OF THE INVENTION. The beneficial effects on proliferative diseases can be determined directly through the results of these studies or by changes in the study design which are known as such to a person skilled in the art. Such studies are, in particular, suitable to compare the effects of a monotherapy using the active ingredients and a COMBINATION OF THE INVENTION.
Preferably, the combination partner (a) is administered with a fixed dose and the dose of the combination partner (b) is escalated until the Maximum Tolerated Dosage (MTD) is reached. The efficacy of the treatment can be determined in such studies, e.g., after 18 or 24 weeks by radiologic evaluation of the tumors every 6 weeks.

Alternatively, a placebo-controlled, double blind study can be used in order to prove the benefits of the COMBINATION OF THE INVENTION mentioned herein.

In a dose escalation study, preferably, the HER-1 or HER-2 antibody, e.g. trastuzumab, is administered in a fixed dose, preferably, 2mg/kg trastuzumab administered as a 30 minute infusion, e.g. on a once weekly basis, and the dose of the epothilone derivative of formula I, e.g. epothilone B, is escalated. In a preferred embodiment of the study, the epothilone derivative of formula I is administered once weekly i.v. for three weeks, followed by one week off starting, e.g., with 0.5 mg/$m^2$ epothilone B and escalating the dose to 1.0 mg/$m^2$, 1.5 mg/$m^2$, 2.0 mg/$m^2$ and 2.5 mg/$m^2$ until the Maximum Tolerated Dosage is reached. Each four week interval will be considered one cycle. The efficacy of the treatment can be determined in these studies, e.g., after 18 or 24 weeks by radiological evaluation of the tumors every 6 weeks. In one embodiment of such a dose escalating clinical study, a HER-1 or a HER-2 antibody is given as a pretreatment. Pretreatment means that before the treatment with the COMBINATION OF THE INVENTION is started, the HER-1 or HER-2 antibody is administered to the patient, preferably as a 90-minute infusion with a weekly dose of about 4mg/kg, for a period of about one or two weeks.

The response criteria relating to the evaluation of target lesions to be applied in a clinical study, e.g. a breast cancer study, as described above are defined as follows:

Complete Response (CR): disappearance of all target lesions.
Partial Response (PR): at least a 30% decrease in the sum of the longest diameter (LD) of target lesions, taking as reference the baseline sum LD.
Stable Disease (SD): Neither sufficient shrinkage to qualify for PR nor sufficient increase to qualify for PD, taking as reference the smallest sum LD since the treatment started. Progressive Disease (PD): at least a 20% increase in the sum of the LD of target lesions, taking as reference the smallest sum LD recorded since the treatment started or the appearance of one or more new lesions.

It is one objective of the present invention to provide a method of treatment of a proliferative disease applying a COMBINATION OF THE INVENTION having a higher rate in CR, PR or SD compared to monotherapy applying only one combination partner.

The COMBINATION OF THE INVENTION can also be applied in combination with surgical intervention, mild prolonged whole body hyperthermia and/or irradiation therapy.

One embodiment of the invention relates to the use of a COMBINATION OF THE INVENTION for the treatment of or for the preparation of a medicament for the treatment of a solid tumor disease.

It is a further objective of this invention to provide a pharmaceutical composition comprising a quantity, which is jointly therapeutically effective against a proliferative disease comprising the COMBINATION OF THE INVENTION. In this composition, the combination partners (a) or (b), and (c) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

The novel pharmaceutical composition contains, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In preparing the compositions for oral dosage form, any of the usual pharmaceutical media may be employed, such as, for example, water, glycols, oils, alcohols, flavoring agents, preservatives, coloring agents; or carriers such as starches, sugars, microcristalline cellulose, diluents, granulating agents, lubricants, binders, disintegrating agents and the like in the case of oral solid preparations such as, for example, powders, capsules and tablets, with the solid oral preparations being preferred over the liquid preparations.

In particular, a therapeutically effective amount of each of the combination partner of the COMBINATION OF THE INVENTION may be administered simultaneously or sequentially and in any order, and the components may be administered separately or as a fixed combination. For example, the method of treatment of a proliferative disease according to the invention may comprise (i) administration of the first combination partner in free or pharmaceutically acceptable salt form and (ii) adminstration of the second combination partner in free or pharmaceutically acceptable salt form, simultaneously or sequentially in any order, in jointly therapeutically effective amounts, preferably in synergistically effective amounts, e.g. in daily or weekly dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen for the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise.

If the the warm-blooded animal is a human, the dosage of a compound of formula I is preferably in the range of about 0.25 to 75, preferably 0.5 to 50, e.g. 2.5, $mg/m^2$ once weekly for two to four, e.g. three, weeks, followed by 6 to 8 days off in the case of an adult patient. In one embodiment of the invention, epothilone B is administered in accordance with the treatment schedule described in US 6,302,838 which disclosure is enclosed herein by reference.

Epothilone B is preferably administered in a dose which is calculated according to the formula (III)

$$\text{single dose (mg/m2)} = (0.1 \text{ to } y) \times N \qquad \text{(III)}$$

wherein N is the number of weeks between treatments and y is 6, wherein epothilone B is administered in more than one treatment cycle after an interval of one week to six weeks after the preceding treatment.

In one preferred embodiment of the invention, epothilone B is administered weekly in a dose that is between about 0.1 to 6 $mg/m^2$, preferably between 0.1 and 3 $mg/m^2$, e.g. 2.5 or 3.0 $mg/m^2$, for three weeks after an interval of one to six weeks, especially an interval of one week, after the preceding treatment. In another embodiment of the invention said epothilone B is preferably administered to a human every 18 to 24 days in a dose that is between about 0.3 and 12 $mg/m^2$.

Vinblastine may be administered to a human in a dosage range varying from about 1.5 to 10 $mg/m^2$day.

Vincristine sulfate may be administered parenterally to a human in a dosage range varying from about 0.025 to 0.05 mg/kg body weight * week.

Vinorelbine may be administered to a human in a dosage range varying from about 10 to 50 $mg/m^2$day.

Etoposide phosphate may be administered to a human in a dosage range varying from about 25 to 115 $mg/m^2$day, e.g. 56.8 or 113.6 $mg/m^2$day.

Teniposide may be administered to a human in a dosage range varying from about 75 to 150 mg about every two weeks.

Doxorubicin may be administered to a human in a dosage range varying from about 10 to 100 $mg/m^2$day, e.g. 25 or 50 $mg/m^2$day.

Epirubicin may be administered to a human in a dosage range varying from about 10 to 200 $mg/m^2$day.

Idarubicin may be administered to a human in a dosage range varying from about 0.5 to 50 $mg/m^2$day.

Mitoxantrone may be administered to a human in a dosage range varying from about 2.5 to 25 $mg/m^2$day.

Topotecan may be administered to a human in a dosage range varying from about 1 to 5 $mg/m^2$day.

Irinotecan may be administered to a human in a dosage range varying from about 50 to 350 $mg/m^2$day.

Bicalutamide may be administered to a human in a dosage range varying from about 25 to 50 $mg/m^2$day.

Fadrozole may be administered orally to a human in a dosage range varying from about 0.5 to about 10 mg/day, preferably from about 1 to about 2.5 mg/day

Exemestane may be administered orally to a human in a dosage range varying from about 5 to about 200 mg/day, preferably from about 10 to about 25 mg/day, or parenterally from about 50 to 500 mg/day, preferably from about 100 to about 250 mg/day. If the drug shall be administered in a separate pharmaceutical composition, it can be administered in the form disclosed in GB 2,177,700.

Formestane may be administered parenterally to a human in a dosage range varying from about 100 to 500 mg/day, preferably from about 250 to about 300 mg/day.

Anastrozole may be administered orally to a human in a dosage range varying from about 0.25 to 20 mg/day, preferably from about 0.5 to about 2.5 mg/day.

Letrozole may be administered orally to a human in a dosage range varying from about 1.0 to 2.5 mg/day, e.g. about 2.5 mg/day.

Aminogluthemide may be administered to a human in a dosage range varying from about 200 to 500 mg/day.

Tamoxifen citrate may be administered to a human in a dosage range varying from about 10 to 40 mg/day.

Furthermore, the present invention pertains to the use of a COMBINATION OF THE INVENTION for the treatment of a proliferative disease and for the preparation of a medicament for the treatment of a proliferative disease.

Furthermore, combinations are preferred which comprise (a) at least one antineoplastic agent selected from the group consisting of aromatase inhibitors and antiestrogens and (b) an epothilone derivative of formula I wherein A represents $NR_N$, wherein $R_N$ is hydrogen or lower alkyl, or, more preferably O, R is hydrogen or lower alkyl, most preferably methyl, R' is methyl or methylthio, and Z is a bond, or, preferably, O.

In the compound of formula I preferably A represents O, R is lower alkyl, e.g. ethyl or, most preferably, methyl, R' is methyl or methylthio, Z is preferably O.

One further aspect of the present invention is the use of (a) a HER-1 or a HER-2 antibody or (b) at least one antineoplastic agent selected from the group consisting of aromatase inhibitors, antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors and S-adenosylmethionine decarboxylase inhibitors; in combination with (c) an epothilone derivative of formula I

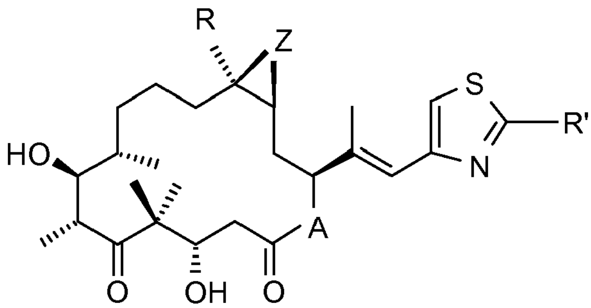

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O or a bond, for the preparation of a medicament for the treatment of a proliferative disease.

In one preferred embodiment of the invention the COMBINATION OF THE INVENTION comprises HERCEPTIN™ and epothilone B.

The COMBINATION OF THE INVENTION can be a combined preparation or a pharmaceutical composition.

Moreover, the present invention relates to a method of treating a warm-blooded animal having a proliferative disease comprising administering to the animal a COMBINATION OF THE INVENTION in a quantity which is jointly therapeutically effective against a proliferative disease and in which the combination partners can also be present in the form of their pharmaceutically acceptable salts.

Furthermore, the present invention pertains to the use of a COMBINATION OF THE INVENTION for the treatment of a proliferative disease and for the preparation of a medicament for the treatment of a proliferative disease.

Moreover, the present invention provides a commercial package comprising as active ingredients COMBINATION OF THE INVENTION, together with instructions for simultaneous, separate or sequential use thereof in the treatment of a proliferative disease.

In one embodiment of the invention, an antidiarrheal agent is administered together with the COMBINATION OF THE INVENTION in order to prevent, control or eliminate diarrhea that is sometimes associated with the administration of epothilones, especially epothilone B. Thus, the present invention also relates to a method of preventing or controlling diarrhea associated with administering an epothilone derivative of formula I, which comprises administering an effective amount of an antidiarrheal agent to the patient receiving treatment with the COMBINATION OF THE INVENTION. Antidiarrheal agents and protocols for their administration are known to those skilled in the art. Antidiarrheal agents suitable for use in the inventive methods and compositions include, but are not limited to, natural opiods, such as tincture of opium, paregoric, and codeine, synthetic opioids, such as diphenoxylate, difenoxin and loperamide, bismuth subsalicylate, octreotide, e.g. as marketed under the trademark SANDOSTATIN™, motilin antagonists and traditional antidiarrheal remedies, such as kaolin, pectin, berberine and muscarinic agents. In particular loperamide or SANDOSTATIN™ can be applied in accordance to the inforamtion provided with the package insert.

## Claims

1. A combination which comprises (a) a HER-1 or a HER-2 antibody or (b) at least one antineoplastic agent selected from the group consisting of aromatase inhibitors antiestrogens, topoisomerase I inhibitors, topoisomerase II inhibitors, microtubule active agents, protein kinase C inhibitors, anti-angiogenic compounds, gonadorelin agonists, antiandrogens, histone deacetylase inhibitors, and S-adenosylmethionine decarboxylase inhibitors and (c) an epothilone derivative of formula I

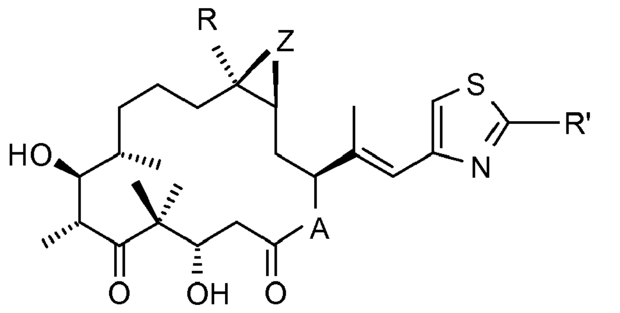

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O,
in which the active ingredients (a) and (b) are present in each case in free form or in the form of a pharmaceutically acceptable salt and optionally at least one pharmaceutically acceptable carrier; for simultaneous, separate or sequential use.

**2.** A combination which comprises

(a) a HER-1 or a HER-2 antibody and
(b) an epothilone derivative of formula I

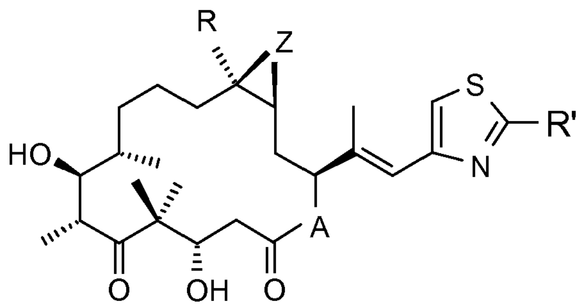

wherein A represents O or $NR_N$, wherein $R_N$ is hydrogen or lower alkyl, R is hydrogen or lower alkyl, R' is methyl and Z is O,
in which the active ingredients (a) and (b) are present in each case in free form or in the form of a pharmaceutically acceptable salt and optionally at least one pharmaceutically acceptable carrier; for simultaneous, separate or sequential use.

**3.** A combination which comprises (a) at least one antineoplastic agent selected from the group consisting of topoisomerase I inhibitors, topoisomerase II inhibitors, microtubule active agents, protein kinase C inhibitors, anti-angiogenic compounds, gonadorelin agonists, anti-androgens, histone deacetylase inhibitors, and S-adenosylmethionine decarboxylase inhibitors and (b) an epothilone derivative of formula I

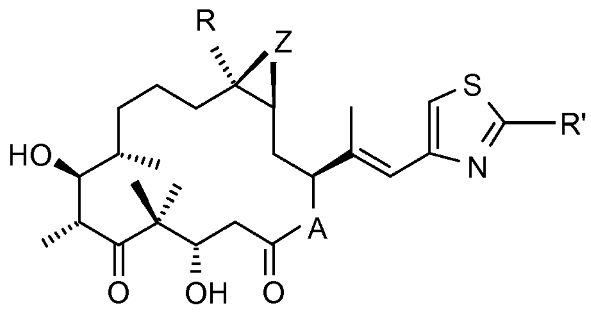

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O,
in which the active ingredients (a) and (b) are present in each case in free form or in the form of a pharmaceutically acceptable salt and optionally at least one pharmaceutically acceptable carrier; for simultaneous, separate or sequential use.

**4.** A combination which comprises (a) at least one antineoplastic agent selected from the group consisting of aromatase inhibitors and antiestrogens and (b) an epothilone derivative of formula I

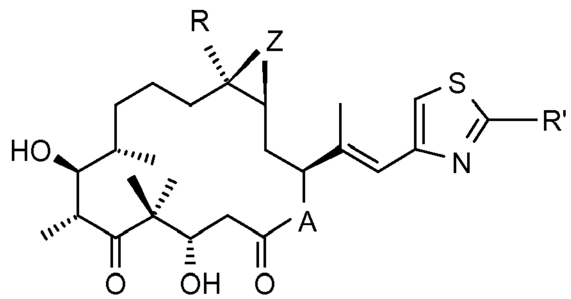

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O,
in which the active ingredients (a) and (b) are present in each case in free form or in the form of a pharmaceutically acceptable salt and optionally at least one pharmaceutically acceptable carrier; for simultaneous, separate or sequential use.

**5.** Combination according to claim 1 which comprises (a) a HER-1 or a HER-2 antibody or (b) at least one antineoplastic agent selected from the group consisting of aromatase inhibitors antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors, and S-adenosylmethionine decarboxylase inhibitors and (c) an epothilone derivative of formula I wherein A represents O or $NR_N$, wherein $R_N$ is hydrogen or lower alkyl, R' is methyl or methylthio, R is hydrogen or lower alkyl, and Z is O,
in which the active ingredients (a) and (b) are present in each case in free form or in the form of a pharmaceutically acceptable salt and optionally at least one pharmaceutically acceptable carrier; for simultaneous, separate or sequential use.

**6.** Combination according to claim 1,2 or 5 wherein the HER-1 or HER-2 antibody is trastuzumab.

**7.** Combination according to claim 1, 3 or 5 wherein the antineoplastic agent is a topoisomerase I inhibitor.

**8.** Combination according to claim 1, 3 or 5 wherein the antineoplastic agent is a topoisomerase II inhibitor.

**9.** Combination according to claim 1, 4 or 5 wherein the antineoplastic agent is an aromatase inhibitor.

**10.** Combination according to claim 1, 3 or 5 wherein the antineoplastic agent is a microtubule active agent.

**11.** Combination according to claim 1 to 10 wherein the epothilone derivative is epothilone B.

**12.** Combination according to any one of claims 1 to 11 which is a combined preparation

**13.** Method of treating a warm-blooded animal having a proliferative disease comprising administering to the animal a combination according to any one of claims 1 to 9 in a quantity which is jointly therapeutically effective against a proliferative disease and in which the compounds can also be present in the form of their pharmaceutically acceptable salts.

**14.** A pharmaceutical composition comprising a quantity which is jointly therapeutically effective against a proliferative disease of a combination according to any one of claims 1 to 12 and at least one pharmaceutically acceptable carrier.

**15.** A combination according to any one of claims 1 to 12 for use in the treatment of a proliferative disease.

**16.** Use of a combination according to any one of claims 1 to 12 for the preparation of a medicament for the treatment of a proliferative disease.

**17.** Use according to claim 15 or 16 wherein the proliferative disease is a solid tumor disease.

**18.** Use of (a) a HER-1 or a HER-2 antibody or (b) at least one antineoplastic agent selected from the group consisting of aromatase inhibitors antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors, and S-adenosylmethionine decarboxylase inhibitors in combination with (c) an epothilone derivative of formula I

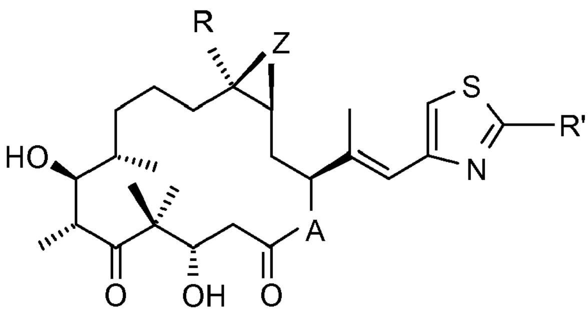

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O,
for the preparation of a medicament for the treatment of a proliferative disease.

**19.** A commercial package comprising (a) a HER-1 or a HER-2 antibody or (b) at least one antineoplastic agent selected from the group consisting of aromatase inhibitors antiestrogens, topoisomerase I inhibitors, topoisomerase II inhibitors, microtubule active agents, protein kinase C inhibitors, anti-angiogenic compounds, gonadorelin agonists, anti-androgens, histone deacetylase inhibitors, and S-adenosylmethionine decarboxylase inhibitors and (c) an epothilone derivative of formula I

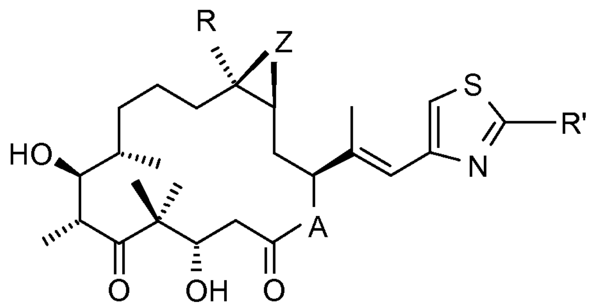

(I)

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O,
together with instructions for simultaneous, separate or sequential use thereof in the treatment of a proliferative disease.

Europäisches Patentamt
European Patent Office
Office européen des brevets

## PARTIAL EUROPEAN SEARCH REPORT

which under Rule 63 of the European Patent Convention shall be considered, for the purposes of subsequent proceedings, as the European search report

Application Number
EP 09 17 8302

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/01124 A (SLOAN-KETTERING INSTITUTE FOR CANCER RESEARCH)<br>14 January 1999 (1999-01-14)<br>* claims 1,3,4,45-47 *<br>----- | 1,3-5,8,<br>10-16,18 | INV.<br>A61K45/06<br>A61K39/395<br>A61K31/426<br>A61K31/427<br>A61P35/00 |
| X | WO 00/47584 A (SCHERING)<br>17 August 2000 (2000-08-17)<br>* claim 1 *<br>* page 59 - page 60 *<br>----- | 1,3-5,7,<br>8,10-19 | |
| X | US 2002/058286 A1 (S.J.DANISHEFSKY E.A.)<br>16 May 2002 (2002-05-16)<br>* claim 1 *<br>* page 30, column 1 *<br>* page 31 - page 32 *<br>* page 33, column 1 *<br>----- | 1,3-5,8,<br>10-16,18 | |
| X | WO 01/64650 A (SLOAN-KETTERING INSTITUTE FOR CANCER RESEARCH)<br>7 September 2001 (2001-09-07)<br>* claims 1,11,28,30,33,34 *<br>* page 74, lines 17-28 *<br>* page 78, lines 15-31 *<br>----- | 1,3-5,7,<br>8,10-19 | TECHNICAL FIELDS SEARCHED (IPC)<br>A61K |
| | -/-- | | |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| Munich | 14 January 2010 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 09 17 8302

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/81341 A (SCHERING) 1 November 2001 (2001-11-01) * claims 1,2 * * page 63 - page 64 * ----- | 1,3-5,7, 10,12-19 | |
| X | WO 01/81342 A (SCHERING) 1 November 2001 (2001-11-01) * claim 1 * * page 53 - page 54 * ----- | 1,3-5,7, 10,12-19 | |
| X | WO 01/72721 A (BRISTOL-MYERS SQUIBB) 4 October 2001 (2001-10-04) * claims 1,23-26,29,30 * * page 23, line 36 - page 24, line 4 * ----- | 1-19 | TECHNICAL FIELDS SEARCHED (IPC) |
| P,X | WO 02/072085 A (BRISTOL-MYERS SQUIBB) 19 September 2002 (2002-09-19) * claims 1,7,8,23,26,34,36 * * page 21, line 30 - page 26, line 10 * * page 44 - page 45 * ----- | 1-19 | |

1

EPO FORM 1503 03.82 (P04C10)

Europäisches Patentamt
European Patent Office
Office européen des brevets

INCOMPLETE SEARCH
SHEET C

Application Number
EP 09 17 8302

Although claim 13 is directed to a method of treatment of the human/animal body, the search has been carried out and based on the alleged effects of the compound/composition.

-----

Reason for the limitation of the search (non-patentable invention(s)):

Rule 39.1(iv) PCT - Method for treatment of the human or animal body by therapy

-----

Further limitation of the search

Reason for the limitation of the search:

Present claims 1-5,7-19 relate to a product/compound/method defined by reference to a desirable characteristic or property, namely:
1) "HER-1 antibody"
2) "HER-2 antibody"
3) "Antineoplastic agent"
4) "Aromatase inhibitors"
5) "Antiestrogens"
6) "Topoisomerase I inhibitors"
7) "Topoisomerase II inhibitors"
8) "Microtubule active agents"
9) "Protein kinase C inhibitors"
10) "Anti-angiogenic compounds"
11) "Gonadorelin agonists"
12) "Anti-androgens"
13) " Histone deacetylase inhibitors"
14) "S-adenosylmethionine decarboxylase inhibitors"

The claims cover all products/compounds/methods having this characteristic or property, whereas the application provides support within the meaning of Article 6 PCT and/or disclosure within the meaning of Article 5 PCT for only a very limited number of such products/compounds/methods. In the present case, the claims so lack support, and the application so lacks disclosure, that a meaningful search over the whole of the claimed scope is impossible. Independent of the above reasoning, the claims also lack clarity (Article 6 PCT). An attempt is made to define the product/compound/method by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely the compound described in claim 6 and the compounds described on pages 18-20 of the present application, with due regard to the general idea underlying the present application.

## ANNEX TO THE EUROPEAN SEARCH REPORT ON EUROPEAN PATENT APPLICATION NO. EP 09 17 8302

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2010

| Patent document cited in search report | | Publication date | Patent family member(s) | | | Publication date |
|---|---|---|---|---|---|---|
| WO 9901124 | A | 14-01-1999 | AU | 756699 | B2 | 23-01-2003 |
| | | | AU | 5792998 | A | 25-01-1999 |
| | | | CA | 2273083 | A1 | 14-01-1999 |
| | | | DE | 69734362 | T2 | 20-07-2006 |
| | | | EP | 0977563 | A1 | 09-02-2000 |
| | | | JP | 2001507716 | T | 12-06-2001 |
| | | | TW | 504511 | B | 01-10-2002 |
| WO 0047584 | A | 17-08-2000 | AU | 4101800 | A | 29-08-2000 |
| | | | BG | 105803 | A | 29-03-2002 |
| | | | BR | 0008206 | A | 19-02-2002 |
| | | | CA | 2360952 | A1 | 17-08-2000 |
| | | | CN | 1340053 | A | 13-03-2002 |
| | | | CZ | 20012886 | A3 | 14-11-2001 |
| | | | EE | 200100422 | A | 16-12-2002 |
| | | | EP | 1161430 | A2 | 12-12-2001 |
| | | | HU | 0105330 | A2 | 29-05-2002 |
| | | | JP | 2002536450 | T | 29-10-2002 |
| | | | MX | PA01008148 | A | 21-07-2003 |
| | | | NO | 20013900 | A | 11-10-2001 |
| | | | PL | 350190 | A1 | 18-11-2002 |
| | | | SK | 11452001 | A3 | 04-04-2002 |
| | | | US | 2006040990 | A1 | 23-02-2006 |
| | | | US | 7001916 | B1 | 21-02-2006 |
| US 2002058286 | A1 | 16-05-2002 | NONE | | | |
| WO 0164650 | A | 07-09-2001 | AU | 4337201 | A | 12-09-2001 |
| | | | CA | 2401800 | A1 | 07-09-2001 |
| | | | EP | 1259490 | A2 | 27-11-2002 |
| | | | JP | 2004500388 | T | 08-01-2004 |
| WO 0181341 | A | 01-11-2001 | AU | 5481201 | A | 07-11-2001 |
| | | | DE | 10020899 | A1 | 25-10-2001 |
| | | | EP | 1282618 | A2 | 12-02-2003 |
| | | | JP | 2003531206 | T | 21-10-2003 |
| | | | NO | 20025028 | A | 18-10-2002 |
| | | | US | 2003139460 | A1 | 24-07-2003 |
| WO 0181342 | A | 01-11-2001 | AU | 6222101 | A | 07-11-2001 |
| | | | DE | 10020517 | A1 | 25-10-2001 |
| | | | EP | 1276740 | A2 | 22-01-2003 |
| | | | JP | 2003531207 | T | 21-10-2003 |
| | | | NO | 20025029 | A | 18-10-2002 |
| | | | US | 2004058969 | A1 | 25-03-2004 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 09 17 8302

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2010

| Patent document cited in search report | Publication date | Patent family member(s) | Publication date |
|---|---|---|---|
| WO 0172721 A | 04-10-2001 | AR 028296 A1 | 30-04-2003 |
| | | AT 430570 T | 15-05-2009 |
| | | AU 4768301 A | 08-10-2001 |
| | | AU 2001247683 B2 | 12-01-2006 |
| | | BR 0109517 A | 29-08-2006 |
| | | CA 2404712 A1 | 04-10-2001 |
| | | CN 1419452 A | 21-05-2003 |
| | | CZ 20023220 A3 | 12-11-2003 |
| | | EP 1272193 A2 | 08-01-2003 |
| | | ES 2325055 T3 | 25-08-2009 |
| | | HU 0301690 A2 | 28-08-2003 |
| | | JP 2003528864 T | 30-09-2003 |
| | | MX PA02009463 A | 10-04-2003 |
| | | NO 20024610 A | 25-11-2002 |
| | | UY 26635 A1 | 25-10-2001 |
| | | ZA 200207766 A | 20-01-2003 |
| WO 02072085 A | 19-09-2002 | BG 108137 A | 31-01-2005 |
| | | BR 0207961 A | 20-04-2004 |
| | | CA 2440555 A1 | 19-09-2002 |
| | | CZ 20032376 A3 | 15-09-2004 |
| | | EE 200300440 A | 15-12-2003 |
| | | EP 1383490 A1 | 28-01-2004 |
| | | HR 20030831 A2 | 31-08-2004 |
| | | HU 0400203 A2 | 30-08-2004 |
| | | IS 6950 A | 12-09-2003 |
| | | JP 2004529904 T | 30-09-2004 |
| | | MX PA03008135 A | 12-12-2003 |
| | | NO 20034056 A | 05-11-2003 |
| | | RU 2321400 C2 | 10-04-2008 |
| | | SK 11082003 A3 | 03-08-2004 |
| | | YU 71603 A | 25-05-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9943320 A
- WO 9310121 A
- US 6194181 B
- WO 9825929 A
- WO 9808849 A
- WO 9943653 A
- WO 9822461 A
- WO 0031247 A
- WO 9902514 A
- WO 9967252 A
- WO 0172721 A
- US 4939168 A
- US 5618487 A
- GB 2280677 A
- WO 9824429 A
- US 5789605 A
- US 6031133 A
- US 5093330 A
- WO 9948896 A
- US 4659516 A
- US 5677171 A
- WO 9917804 A
- US 4100274 A
- US 5843901 A
- US 4636505 A
- FR 901228
- WO 0222577 A
- US 5461076 A
- WO 9939694 A
- US 6302838 B
- GB 2177700 A


### Non-patent literature cited in the description


- **Bollag et al.** *Cancer Research,* 1995, vol. 55, 2325-33